# EUROPÄISCHE PATENTANMELDUNG

(11) **EP 3 981 711 A1**
(43) Veröffentlichungstag der Anmeldung: **13.04.2022**
(21) Anmeldenummer: 21201928.5
(22) Anmeldetag: 11.10.2021
(51) Int. Cl.: B65F 1/02, A61L 9/013, B01J 20/22

(54) **KOMPOSTIERBARE, FEUCHTIGKEIT BINDENDE UND GERUCHSHEMMENDE VORRICHTUNG**

(30) Priorität: 10.10.2020 DE 202020105814 U; 17.05.2021 DE 202021102676 U
(71) Anmelder: Ligresa GbR, 69124 Heidelberg (DE)
(72) Erfinder: Liebhardt, Manal, 69124 Heidelberg (DE); Muna, Gremmelmaier, 69124 Heidelberg (DE)
(74) Vertreter: Trösch, Dominique

(57) **Zusammenfassung**

Kompostierbare, feuchtigkeitsbindende und geruchshemmende Vorrichtung (1) mit einem Feuchtigkeit aufnehmenden Füllstoff (3) zur Verwendung als Einlage in Abfallbehältern, aufweisend:
- Eine für Feuchtigkeit durchlässige Wandung (2) bestehend aus einer oberen Wandung (2a) und einer unteren Wandung (2b),
- Bereiche (4), in denen die obere Wandung (2a) und untere Wandung (2b) aufeinandertreffen und miteinander verbunden sind,
- zwischen den Bereichen (4) befindliche Hohlräume (5) mit darin befindlichem Füllstoff (3), dergestalt, dass die Hohlräume (5) mit dem darin befindlichem Füllstoff (3) durch die sie unmittelbar umgebenden Bereiche (4), in denen die obere (2a) und untere Wandung (2b) aufeinandertreffen und miteinander verbunden sind, jeweils begrenzt werden, wobei die Vorrichtung (1) mindestens zwei Hohlräume (5) mit darin befindlichem Füllstoff (3) aufweist, und die Hohlräume in einer Ebene liegen.

## Beschreibung

Die vorliegende Erfindung betrifft eine kompostierbare, Feuchtigkeit bindende und geruchshemmende Vorrichtung.

### Hintergrund der Erfindung

In Haushalten fallen Abfälle, insbesondere Restmüll und Bioabfall, an. Seit geraumer Zeit wird Bioabfall in Privathaushalten separat gesammelt. Das Sammeln und Aufbewahren von Bioabfall dabei oftmals als unhygienisch betrachtet. Grund dafür ist vor allem die im Allgemeinen unmittelbar einsetzende Verrottung organischer Abfälle und die damit einhergehende Geruchs- und Feuchtigkeitsentwicklung. Diese findet bei Entsorgung organischer Abfälle auch im normalen Haushaltsmüll statt. Die Feuchtigkeitsentwicklung kann zur Verschmutzung von Müllbehältern führen, vor allem wenn Müllbeutel undicht sind oder aus nicht feuchtigkeitsbeständigem Material - etwa Papier - bestehen. Die Feuchtigkeitsentwicklung in Haushalts- und/oder Biomüll kann außerdem zu Kondensation von Wasser an Mülleimerdeckeln führen, was von Nutzern wiederum als eklig empfunden werden kann.

Davon abgesehen bietet eine entsprechende Flüssigkeit, die aus verrottendem Haushalts- oder Biomüll austritt, beste Lebensbedingungen für Fliegen und Maden. Sie führt ferner zu einer erhöhten Geruchsbelästigung durch den verrottenden Haushalts- oder Biomüll. Der erhöhte Feuchtigkeitsgehalt führt außerdem dazu, dass die Bildung von Schimmel befördert wird, und dass entsprechend feuchter Müll bei Temperaturen unter 0°C leichter festfriert.

Aus dem Stand der Technik ist bekannt, zur Bindung von Feuchtigkeit und zur Hemmung von einer etwaigen Geruchsbildung Pulver bzw. Streumittel auf mineralischer Basis einzusetzen, etwa auf Basis von Gesteinsmehlen. Die bekannten Streumittel können mit außerdem pflanzlichen Duftstoffen versetzt sein, etwa Geraniol und/oder Lavandinöl, die einem Befall durch Insekten vorbeugen. Ein Nachteil dieser Mittel ist die mit ihnen einhergehende Staubbildung, die eine saubere Handhabung und den damit einhergehenden Einsatz im Haushalt erschwert.

Zur Lösung dieses Problems beschreibt die DE 20 2019 001 010 U1 eine Einlage für Bioabfall-Beutel, die kompostierbar sein soll, das Wachstum von Mikroorganismen sowie unangenehme Geruchsentwicklung hemmen soll wobei bei der Einlage ein Füllmaterial durch eine obere und untere Lage Papier umhüllt ist, die durch eine Klebeschicht miteinander verbunden sind. Das Füllmaterial der dort beschriebenen Einlage besteht zu 90-95 % aus Weizenstroh-Pellets, welche die flüssigkeitsbindende Komponente darstellen, und der restliche Bestandteil besteht zu 5-10 % aus getrocknetem Zitronengras, das eine wachstumshemmende Wirkung auf Bakterien und Pilze aufweisen soll aufgrund seines natürlichen Gehalts an ätherischen Ölen, sowie einer unangenehmen Geruchsentwicklung entgegenwirken soll.

Die Feuchtigkeitsaufnahmefähigkeit dieser Einlage zur Innenbehandlung von Haushalts-Bioabfallbehältern ist aber vergleichsweise eingeschränkt. Das gilt sowohl in Hinblick auf das Feuchtigkeitsaufnahmevermögen derartiger Vorrichtungen, als auch in Hinblick auf die Geschwindigkeit der Feuchtigkeitsaufnahme. Außerdem ist offenbar vergleichsweise viel Zitronengras notwendig, um eine Wirkung der darin enthaltenen ätherischen Öle erzielen zu können.

Unabhängig davon ist ein weiterer Nachteil der vorbeschriebenen Einlage für Bioabfall-Beutel, dass diese einstückig ausgebildet ist. D.h. die in DE 20 2019 001 010 U1 beschriebene Einlage des Standes der Technik besteht aus einer Art rechteckigem Kissen, in dem die Füllung in einer einzigen Kammer enthalten ist. Die angegebenen Maße für die Einlage sind: 12-13 cm lang, 1,5 cm dick und 7 bis 8 cm breit. Ein solches "Kissen" mit lose darin befindlichem Material, bei dem die Umhüllung wasser- und luftdurchlässig und gut kompostierbar ist, hat den Nachteil, dass das Füllmaterial sich verschieben kann, wenn die Einlage in einen Bioabfall-Beutel eingebracht wird.

Will man darüber hinaus eine größere Grundfläche bedecken, wie beispielsweise die Grundfläche einer üblichen Biotonne zur Sammlung von Bioabfall und regelmäßig wiederkehrenden, etwa wöchentlichen Entleerung durch die Müllabfuhr, benötigt man eine Einlage mit größeren Abmessungen. Diese kann man nicht ohne weiteres in eine Biotonne hineinlegen, man muss sie vielmehr auf den Boden der Tonne fallen lassen. Das gelingt aufgrund der Form als Kissen mit lose darin befindlichem Füllmaterial nicht, ohne dass sich das Füllmaterial verschieben würde, was nicht gewünscht ist. Davon abgesehen kommt eine solche Vorrichtung nicht immer plan auf dem Boden einer Abfalltonne zu liegen, weil sie beim hinunterfallen eine andere Orientierung einnehmen kann, gegebenenfalls verkantet, und im Resultat den Boden nicht bedeckt, wie gewünscht, sondern ggf. eine Seitenwandung. Zusätzliche Probleme ergeben sich dann, wenn die Tonne, wie heutzutage allgemein üblich, mit Rädern ausgestattet ist, was zur Folge hat, dass der Innenraum der Tonne sich zum Boden hin dergestalt verjüngt, dass eine Stufe im unteren Bereich des Tonneninnenraums ausgebildet wird, da die Räder und die dazugehörige Achse außerhalb des Innenraums der Tonne unterhalb der besagten Stufe angebracht sind. Der Boden der Tonne befindet sich also erst unterhalb der besagten Stufe. Je nach Tonnenvolumen können hier die Abmessungen außerdem variieren.

Bekannt sind ferner
- aus CN107185021A und CN209528614U die Verwendung von Kokosnusspalmwedelmaterial als Polstermaterial;
- aus CN105557382A die Verwendung von Kokoserde und Bentonit zur Feuchtigkeitsbindung in Gabionen, wobei Kokoserde und Bentonit in einem Stoffbehältnis aus Polypropylen, Polyethylen, Polyester, Glasfaser oder Polyamidmaterial befindlich sind;
- aus CN202376501U geruchshemmende Beutel zur Verwendung in Kühlschränken, enthaltend Aktivkohleblöcke aus Kokosnussschalen als geruchshemmendes Mittel;
- aus CN109200322A ein Luftreinigungsmittel zur Verwendung in Klimaanlagen enthaltend unter anderem Kokosnusspulver (neben anderen Bestandteilen, wie Aktivkohle);
- aus JP2013022293A die Verwendung von Fasern aus Kokosnussschalen als Bestandteil eines geruchshemmenden Materials (in Form von Schüttgut) zur Behandlung von Gülle;
- aus US 10,966,441 B1 Sicherheitsfilter für Kühlvorrichtungen die hauptsächlich mit einem Material aus Diatomeen befüllt sind, das außerdem mit Pflanzenbestandteilen, beispielsweise Kokosnussfasern, vermengt sein kann, wobei diese in einem biologisch abbaubaren Kraftpapier enthalten sein können.

Die im Stand der Technik zur Geruchshemmung eingesetzten Bestandteile der Kokosnusspalme werden im Haushalt also allenfalls im Zusammenhang mit der Geruchshemmung in Kühlschränken beschrieben. Die einzige vorbeschriebene Verwendung im Zusammenhang mit Gülle erfolgt außerhalb eines gewöhnlichen Haushalts und nicht in einer Weise, die im Haushalt handhabbar wäre, ohne dabei Verschmutzungen zu verursachen, nämlich in Form eines losen Schüttguts. Es kommen außerdem ganz unterschiedliche Bestandteile der Kokospalme zum Einsatz.

Zur Überwindung von vorgenannten Nachteilen des Standes der Technik wird bereitgestellt:
Eine kompostierbare, feuchtigkeitsbindende und geruchshemmende Vorrichtung mit einem Feuchtigkeit aufnehmenden Füllstoff zur Verwendung als Einlage in Abfallbehältern, aufweisend:
- Eine für Feuchtigkeit durchlässige Wandung bestehend aus einer oberen Wandung und einer unteren Wandung,
- Bereiche, in denen die obere Wandung und untere Wandung aufeinandertreffen und miteinander verbunden sind,
- zwischen den Bereichen befindliche Hohlräume mit darin befindlichem Füllstoff, dergestalt, dass die Hohlräume mit dem darin befindlichem Füllstoff durch die sie unmittelbar umgebenden Bereiche, in denen die obere und untere Wandung aufeinandertreffen und miteinander verbunden sind, jeweils begrenzt werden, wobei die Vorrichtung mindestens zwei Hohlräume mit darin befindlichem Füllstoff aufweist, und die Hohlräume in einer Ebene liegen.

Die erfindungsgemäße Vorrichtung kann beispielsweise die Form eines Pads oder Kissens mit mehreren Kammern annehmen. Es versteht sich außerdem, dass die Vorrichtung eine flächige Ausdehnung hat, d.h. die Hohlräume liegen in einer Ebene, nicht übereinander.

"Kompostierbar" bedeutet biologisch abbaubar, kompostierbar und/oder industriell kompostierbar.

"Abfallbehälter" bezeichnet jede Art von Behälter zur Aufnahme von Abfall, also Beutel, Tonnen, und sonstige Behälter, insbesondere Haushaltsabfallbehälter, insbesondere in Form von Bioabfallbeuteln, Restabfallbeuteln, Bioabfalltonnen, oder Restabfalltonnen u. ä..

Die "Hohlräume" können gleich oder unterschiedlich dimensioniert und/oder geformt sein. Beispielsweise kann die Vorrichtung von oben betrachtet kreisförmig ausgebildet sein, und zwei jeweils halbkreisförmige Hohlräume aufweisen, oder einen innenliegenden kreisförmigen Hohlraum und mindestens einen diesen umgebenden ringförmigen Hohlraum. Die Vorrichtung kann von oben betrachtet aber auch rechteckig oder quadratisch ausgebildet sein, was bevorzugt ist. Unabhängig davon können die Hohlräume von oben betrachtet rechteckig oder quadratisch ausgebildet sein, was ebenfalls bevorzugt ist, und wie Felder auf einem Schachbrett angeordnet sein, was bevorzugt ist, oder versetzt.

Abfalltonnen, egal ob für Restmüll oder Bioabfall, dienen der Sammlung von Abfällen bevor diese an die Müllabfuhr übergeben werden bzw. von dieser abgeholt werden. Sie sind diesen Anforderungen entsprechend ausgebildet. Insbesondere nehmen sie vergleichsweise große Volumen an Müll bzw. Abfall auf. Welche Tonnengröße und Form verwendet wird, hängt letztlich von den örtlichen Gegebenheiten ab. Üblich sind heutzutage Tonnen mit 120 l, 240 l oder gar 1100 l Volumen, wobei auch andere Volumina möglich sind.

In der vorliegenden Anmeldung werden die Begriffe "Müll" und "Abfall" synonym verwendet.

Dadurch, dass die Vorrichtung mindestens zwei Hohlräume aufweist, die mit einem Feuchtigkeit bindenden und geruchshemmenden Füllstoff befüllt sind, kann der Füllstoff nicht so leicht verrutschen. Der Füllstoff bleibt mit anderen Worten in einer erfindungsgemäßen Vorrichtung gleichmäßiger verteilt, wenn die Vorrichtung angewendet wird, d.h. in einen Abfallbehälter überführt wird.

Die Anzahl der erforderlichen Hohlräume kann den Bedürfnissen entsprechend angepasst werden. Für größere Abfallbehälter bietet sich das Vorsehen einer größeren Anzahl an Hohlräumen an als für kleinere Abfallbehälter. Eine gleichmäßigere Verteilung von Füllstoff wird schon durch das Vorsehen von mindestens zwei Hohlräumen mit Füllstoff bewirkt.

Kompostierbare, geruchshemmende und Feuchtigkeit bindende Füllstoffe sind dem Fachmann bekannt und beispielsweise beschrieben in vorstehend gewürdigten Dokumenten des Standes der Technik.

Bevorzugt weist der Füllstoff mindestens 10%, bevorzugt mindestens 20%, bevorzugter mindestens 50%, noch bevorzugter mindestens 70%, am meisten bevorzugt mindestens 80% Kokoserde, und am allermeisten bevorzugt ist der Füllstoff im Wesentlichen aus Kokoserde.

Bevorzugt besteht der Füllstoff aus Kokoserde.

"Kokoserde" bedeutet hierin nicht gequollene Kokoserde in Pulverform, die zu 100 % aus organischem Kokoshumus aus Kokosfasern besteht. Kokoserde kann aufgrund ihrer besonderen Zellstruktur viel Wasser aufnehmen. Außerdem kann sie infolge der Sterilisierung nicht oder allenfalls erschwert von Schimmel befallen werden.

Kokoserde ist nicht nur in der Lage, viel mehr Wasser aufzunehmen, als Strohpellets, nämlich bis zum zehn-fachen ihres Eigengewichts an Wasser, sondern darüber hinaus kann Kokoserde Feuchtigkeit sehr viel rascher aufnehmen , als Strohpellets vermögen. Andere denkbare Füllstoffe pflanzlichen Ursprungs vermögen demgegenüber weitaus weniger Feuchtigkeit aufzunehmen - beispielsweise Chinaschilffasern (Miscanthus) oder Hanffasern.

Kokoserde zeichnet sich außerdem dadurch aus, dass sie auch nach einer etwaigen Austrocknung in der Lage ist, Wasser leicht wieder aufzunehmen. Diese Eigenschaft unterscheidet Kokosfasern von Torf. Während feuchter Torf zusätzliches Wasser aufnehmen kann, ist trockener Torf vergleichsweise wenig wasseraufnahmefähig.

Solche Vorrichtungen sind folglich besser als Vorrichtungen des Standes der Technik dazu geeignet, in Müllbeuteln oder Biomülltonnen eingesetzt zu werden, um Feuchtigkeit zu binden und entstehende Gerüche zu hemmen. Auf diese Weise kann die erfindungsgemäße Vorrichtung auch einer etwaigen Schimmelbildung gut entgegenwirken. Der Füllstoff Kokoserde sorgt für eine besonders rasche Kompostierbarkeit der Vorrichtung. Im Wesentlichen muss nämlich nur das Material der Pad- bzw. Kissenhülle oder der Vorrichtung verrotten.

Kokoserde ist auch deshalb vorteilhaft, weil sie im Herstellungsprozess thermisch sterilisiert wird, und frei von Samen und unerwünschten Organismen ist. Die Sterilität der Kokoserde wirkt Schimmelbildung zusätzlich entgegen. Sie ist außerdem förderlich für die Qualität des aus dem Verrottungsprozess entstehenden Kompostierproduktes. Insbesondere sind die in der Kokoserde noch enthaltenen Pflanzenfasern einerseits sehr wasseraufnahmefähig, andererseits aber auch strukturfest. Das Vorsehen von Kokoserde als Bestandteil eines Kompostiergutes ist auch aus diesem Grund förderlich für die Qualität des Kompostierproduktes, da mit Kokoserde versetzter Kompost weniger leicht verdichtet. Schließlich wird Kokoserde aus nachwachsenden Rohstoffen gewonnen und ist vollständig biologisch abbaubar.

Es ist ferner bevorzugt, dass die obere Wandung und die untere Wandung in den Bereichen, in denen die obere Wandung und untere Wandung aufeinandertreffen und miteinander verbunden sind, mittels Heißversiegelung oder Kleben miteinander verbunden sind.

Dies hat folgenden Hintergrund: Die Hülle erfindungsgemäßer Vorrichtungen kann aus kompostierbarem Filterpapier oder kompostierbarem Zellstoff bestehen. Entsprechende Vorrichtungen können hergestellt werden, indem ein Füllstoff, z.B. Kokoserde, mit Insekten vertreibenden Mitteln behandelt oder unbehandelt, in eine Filtertüte aus Zellstoff gefüllt wird, die danach verschlossen, beispielsweise an dem offenen Randbereich und in den Bereichen, in denen die obere Wandung und untere Wandung aufeinandertreffen und miteinander verbunden sind, zwischen mindestens zwei Hohlräumen verpresst wird. Der Füllstoff, z. B. Kokoserde kann auch einfach zwischen zwei Zellstoffblättern eingebracht sein, wobei die Zellstoffblätter an ihrem jeweiligen Rand und in den Bereichen zwischen mindestens zwei Hohlräumen direkt aufeinander zu liegen kommen und miteinander verbunden bzw. verpresst sind. Ein entsprechendes Verpressen kann mittels Rändeln erfolgen. Dadurch soll die Verbindung stabilisiert werden und die Druckbeständigkeit des Pad- bzw. Kissenverschlusses gesteigert werden.

In der Praxis gestaltet sich diese Verschlussmethode insofern als schwierig, da keine Maschine existiert, um das Produkt in großen Mengen herzustellen. Weiterhin ist die Methode mit bisher vorhandenen Maschinenwerkzeugen nicht immer ausreichend stabil, so hergestellte Pads können bei jetzigem technischen Stand, bei der Handhabung an den Fügeflächen leicht aufreißen.

Heißversiegeln oder Kleben sorgt für eine stabilere Verbindung. Die Tatsache, dass die Vorrichtung kompostierbar ist, bedeutet, dass kompostierbare Heißversiegelungsmittel bzw. Klebstoffe zum Einsatz kommen.

"Heißversiegelungsmittel" bedeutet in diesem Zusammenhang ein schmelzfähiges Material, dass zur Heißversiegelung verwendet werden kann. Heißversiegelung ist ein etabliertes Verfahren, bei dem ein schmelzfähiges Material durch erwärmen geschmolzen wird und dort zum Erstarren oder zur Verfestigung gebracht wird, wo eine Versiegelung stattfinden soll. In diesem Zusammenhang werden typischerweise mit Heißversiegelungsmittel ausgerüstete Materialien verwendet. Biologisch abbaubare Heißversiegelungsmittel sind hinlänglich bekannt, etwa Polymilchsäure bzw. Polylactid (PLA). Beispielsweise werden heißsiegelfähige Papiere zur Herstellung von Teebeuteln damit ausgestattet, oder Teebeutel sogar ganz daraus hergestellt (entsprechende Materialien werden von der Fa. Ahlstrom-Munksjö unter den Bezeichnungen Bioweb^{®}, Bioweb Robust^{®}, Fiber+ vertrieben, bzw. von der Fa. Glatfelter unter der Bezeichnung Dynatec^{™}).Die Verwendung biologisch abbaubarer Heißversiegelungsmittel gewährleistet die Kompostierbarkeit erfindungsgemäßer Vorrichtungen.

PLA kann auf Basis nachwachsender Rohstoffe erzeugt werden. Die biologische Abbaubarkeit und Kompostierbarkeit von PLA in Kombination mit der Erzeugbarkeit aus nachwachsenden Rohstoffen bedeutet, dass es in Form einer erneuerbaren Biomasse eingesetzt werden kann, was besonders bevorzugt ist.

Durch Heißversiegelung verschlossene Vorrichtungen erweisen sich als stabiler, als lediglich durch Verpressen oder mit wasserlöslichem Stärkekleber verschlossene Vorrichtungen. Klebstoffe können Lösungsmittel enthalten, was die Handhabung im Zusammenhang mit biologisch abbaubaren Wandungsmaterialien erschweren kann. So kann es sich als nachteilhaft erweisen, wenn sich eine Klebeschicht auf Stärke-Wasser-Basis bei Flüssigkeitskontakt auflöst, oder ein Papierwerkstoff bereits bei der Herstellung durch einen Kleber, beispielsweise auf Stärke-Wasser-Basis, bereits nass wird. Heißversiegelung ist daher bevorzugt.

Es ist ferner bevorzugt, dass der Füllstoff und/oder die obere Wandung und/oder die untere Wandung mit einem Insekten abwehrenden Mittel, bevorzugt einem natürlichen oder naturidentischen Duftstoff, besonders bevorzugt Geraniol, behandelt ist bzw. sind.

Als Repellentien bzw. Insekten abwehrende Mittel kommen dabei die für einen Fachmann üblichen Stoffe und Verbindungen infrage. Beispielhaft erwähnt seien an dieser Stelle ätherische Öle, aromatische Süßgräser, Basilikum, Bergamotte, Eukalyptus, Geraniol, Ingweröl, Knoblauch, Lavandienöl, Lavendel, Lorbeeröl, Neemöl, Neempulver, Nelkenöl, Palmarosa, Pfefferminzöl, Pyrethrum, Salbei, Teebaumöl, Zedernöl, Zitronella, Zitroneneukalyptus (Citriodiol).

Bevorzugt werden allerdings naturidentische oder aus natürlichen Quellen gewonnene Aromastoffe, beispielsweise Neemöl, Lavendel- / Lavandinöl oder Geraniol.

Durch den Einsatz von Insekten abwehrenden Mitteln bzw. Repellentien in konzentrierter Form wird es nicht notwendig, große Mengen an natürlichen Materialien, die vergleichsweise geringe Mengen an Repellentien, wie etwa ätherischen Ölen, enthalten, einzusetzen. So ist es bei dem erfindungsgemäßen Pad durch den Einsatz von entsprechenden Mitteln nicht notwendig, beispielsweise Zitronengras in einem Gewichtsanteil von 5-10 % vorzusehen, wie im o. a. Stand der Technik.

Durch die Verwendung von naturidentischen oder aus natürlichen Quellen gewonnenen, Insekten vertreibenden Duftstoffen, wie beispielsweise Geraniol, wird erreicht, dass die Kompostierbarkeit der erfindungsgemäßen Pads nicht beeinträchtigt wird, während die Pads zugleich einen Befall von Hausmüll oder Biomüll mit Insekten entgegenwirken.

Bevorzugt weisen erfindungsgemäße Vorrichtungen in jedem Hohlraum (5) mindestens 1, 1,5, 5, 10, 20, 30, 40 oder 50 g Füllstoff auf.

Die Menge kann davon abhängig gemacht werden, für welches Abfallbehältervolumen die Vorrichtung vorgesehen sein soll, und von der Zahl der vorgesehenen Hohlräume. Mehr Hohlräume bewirken eine noch gleichmäßigere Verteilung des Füllstoffs.

Beim Füllstoff können außerdem unterschiedliche Dichten eine Rolle spielen. Beispielsweise weist eine sehr fein vermahlene Kokoserde eine größere Dichte auf, als eine weniger fein vermahlene Kokoserde. Es kann vorgesehen werden, in manchen Hohlräumen Füllmaterial mit einer höheren Dichte einzusetzen, als in anderen Hohlräumen.

Bevorzugt sind erfindungsgemäße Vorrichtungen, bei denen die obere Wandung und/oder die untere Wandung aus einem Material besteht, dass aus einem oder mehreren der folgenden ausgewählt ist: Filterpapier, Vlies, Stoff, Filz, oder aus einem Netz aus biologisch abbaubarem, synthetischem und heißsiegelfähigem Kunststoff.

Bevorzugt sind ferner erfindungsgemäße Vorrichtungen, bei denen die obere Wandung und/oder die untere Wandung aus einem Material besteht, dass aus einem langfaserigen Filterpapier besteht, dass mit einem biologisch abbaubarem Heißversiegelungsmittel, bevorzugt PLA, ausgerüstet ist.

Bevorzugt sind auch erfindungsgemäße Vorrichtungen, bei denen die Vorrichtung in den Hohlräumen einen weiteren Feuchtigkeit aufnehmenden Füllstoff in Form von Zellstoffwatte und/oder einem anderen biologisch abbaubarem bzw. kompostierbarem Material enthält.

Watte bzw. andere biologisch abbaubare, kompostierbare und/oder industriell kompostierbare Fasern nehmen die Flüssigkeit schnell auf und leiten diese an die Kokoserde weiter, wo sie nachhaltig gebunden wird. Da der Sättigungsgrad bei der Kokoserde höher ist, als bei den Fasern bleibt die Flüssigkeit in der Kokoserde gebunden. Außerdem wirkt Kokoserde auch hier einer Schimmelbildung entgegen.

Für kleinere Gebinde bietet es sich aus Kostengründen an, Pads bzw. Vorrichtungen ohne zusätzliche Mittel vorzusehen. Für größere Gebinde können die Pads wie vorstehend beschrieben vorteilhaft mit weiteren saugfähigen Mitteln ausgerüstet werden.

Bevorzugt weisen erfindungsgemäße Vorrichtungen außerdem zwei bis 30, zwei bis 16, oder zwei bis neun Hohlräume auf.

Die Menge kann davon abhängig gemacht werden, für welches Abfallbehältervolumen die Vorrichtung vorgesehen sein soll. Mehr Hohlräume bewirken eine noch gleichmäßigere Verteilung des Füllstoffs.

Als zweckmäßig erweisen sich
- für 120 l Tonnen: Erfindungsgemäße Vorrichtungen mit 9 Hohlräumen,
- für 240 l Tonnen: Erfindungsgemäße Vorrichtungen mit bis zu 16 Hohlräumen,
- für 1100 l Tonnen (die etwa in Mietshäusern zum Einsatz kommen): Erfindungsgemäße Vorrichtungen mit bis zu 30 Hohlräumen.

Bevorzugt ist ferner, dass die erfindungsgemäße Vorrichtung an ihrem Rand eine höhere Flächendichte aufweist, als in ihrem Innenbereich.

Dies kann auf unterschiedliche Weise realisiert werden, etwa durch das Vorsehen unterschiedlich schwerer Bereiche in den Wandungen der Vorrichtung. Einfacher bewerkstelligen lässt sich dies aber durch das Vorsehen von unterschiedlich schweren Hohlräumen.

Sind nur zwei Hohlräume vorgesehen, lässt sich eine entsprechende Vorrichtung z.B. dadurch realisieren, dass der äußere Hohlraum schlauchringförmig um den inneren Hohlraum herum angeordnet ist.

Bevorzugt sind bei einer entsprechenden Vorrichtung mindestens neun Hohlräume vorgesehen, die in Form eines Rechtecks oder Quadrats angeordnet sind, und von denen acht äußere Hohlräume sind, die um einen inneren Hohlraum herum angeordnet sind, wobei die äußeren Hohlräume jeweils ein größeres Füllstoffgewicht aufweisen als der innere Hohlraum.

Es kann im Übrigen in einem inneren Hohlraum Füllstoff mit einer niedrigeren Dichte eingesetzt werden, wie beispielsweise weniger fein vermahlene Kokoserde, als in einem äußeren Hohlraum. Bei inneren und äußeren Hohlräumen mit gleichen Volumina ermöglicht dies ein identisches Füllvolumen der jeweiligen Hohlräume trotz unterschiedlichen Dichten.

Eine höhere Flächendichte am Rand als im Innenbereich einer erfindungsgemäßen Vorrichtung bewirkt, , dass die Vorrichtung beim Einbringen in einen Abfallbehälter so nach unten fällt, dass sie den Boden des Abfallbehälters bedeckt, ohne auf dem Weg dorthin zu verkanten.

In einer bevorzugten Ausführungsform werden erfindungsgemäße Vorrichtungen in Form eines Bandes bereitgestellt, mit einer Vielzahl von längs des Bandes aneinander gereihten erfindungsgemäßen Vorrichtungen, wobei die Bereiche, in denen die obere Wandung und untere Wandung aufeinandertreffen und miteinander verbunden sind ,Teilbereiche umfassen, die sich quer zur Längsrichtung des Bandes erstrecken.

Die Teilbereiche ermöglichen ein Abtrennen erfindungsgemäßer Vorrichtungen von dem Band, um diese zu vereinzeln und Ihrer Zweckbestimmung zuführen zu können.

Bei dieser Ausführungsform sind die sich quer zur Längsseite des Bandes erstreckenden Teilbereiche und gegebenenfalls weitere zwischen Hohlräumen befindliche Bereiche bevorzugt mit Sollbruchstellen versehen.

Auf diese Weise lassen sich erfindungsgemäße Vorrichtungen von dem Band entlang einer Sollbruchstelle abtrennen. Es können außerdem Sollbruchstellen vorgesehen sein, die es gestatten einzelne Hohlräume abzutrennen. Auf diese Weise können die Größen der Vorrichtungen individuell angepasst werden.

Zur Lösung der Probleme des Standes der Technik wird ferner ein Verfahren zur Vermeidung von übermäßiger Geruchsbildung und/oder Bindung von Feuchtigkeit in einem Abfallbehälter, bei dem der Abfallbehälter mit einer erfindungsgemäßen Vorrichtung ausgestattet wird bzw. ist.

Erfindungsgemäße Vorrichtungen können außerdem bereits in erfindungsgemäßen Abfallbehältern vorgesehen sein, beziehungsweise Abfallbehälter können damit ausgerüstet sein. Das bedeutet, dass erfindungsgemäß außerdem ein Abfallbehälter vorgesehen ist, beispielsweise ein Müllbeutel, in dem sich bereits eine erfindungsgemäße Vorrichtung befindet, sodass der Nutzer einen Abfallbehälter unmittelbar benutzen kann, der einerseits geruchshemmend ist, und andererseits Feuchtigkeit bindend ist.

Bevorzugt ist ein erfindungsgemäßer Abfallbehälter, wobei die Vorrichtung an einer beliebigen Wandung, an einer Bodenwandung und/oder an einer seitlichen Wandung des Abfallbehälters befestigt ist.

Wenn mindestens 9 Hohlräume vorgesehen sind, können diese in der Draufsicht auf die Vorrichtung in Form eines Rechtecks oder Quadrates von 3x3 Hohlräumen angeordnet sein, die - ebenfalls in der Draufsicht -jeweils rechteckig oder quadratisch sind.

Erfindungsgemäße Vorrichtungen mit einer kleineren Füllmenge an Füllstoff sind besonders für kleinere Behälter wie etwa kleinere Müllbeutel oder Sammelbehälter geeignet, während erfindungsgemäße Vorrichtungen mit einer größeren Füllmenge an Füllstoff für Müllsammelbehälter mit einem größeren Volumen geeignet sind. Beispiele für Müllsammelbehälter mit größerem Volumen sind Mülltonnen mit einem Volumen von beispielsweise 120-300 l, wie sie von Müllabfuhren in regelmäßigen Abständen geleert werden. Da vor allem in größeren Müllsammelbehältern der Müll länger vorgehalten wird, ist bevorzugt die Vorrichtung mit der größeren Füllmenge mit Geraniol behandelt.

In den nachstehend erörterten Figuren und Beispielen sind die Hohlräume in Draufsicht betrachtet rechteckig oder quadratisch ausgebildet, wobei die Rechtecke oder Quadrate jeweils parallel und in gleichen Abständen zueinander ausgerichtet sind. Eine solche Anordnung ist allgemein bevorzugt und vorteilhaft, weil auf diese Weise zwischen mehreren Hohlräumen liegende Bereiche ausgebildet werden, in denen die erfindungsgemäßen Vorrichtungen flexibel sind, und sich infolgedessen an dieser Stelle besonders gut einer Stufe oder Biegung am Boden eines Abfallbehälters anpassen können. Insbesondere sind die Vorrichtungen dann um eine gedachte Gerade herum beweglich, die sich entlang eines zwischen Hohlräumen liegenden Bereiches erstreckt.

Das bedeutet aber nicht, dass die Hohlräume grundsätzlich immer die vorgenannte Form und Anordnung aufweisen müssen. Insbesondere ist denkbar, dass erfindungsgemäße Vorrichtungen anders angeordnete und geformte Hohlräume aufweisen. Es können innerhalb einer erfindungsgemäßen Vorrichtung auch verschieden ausgebildete Hohlräume, d.h. Hohlräume mit unterschiedlichen Formen und Volumina, enthalten sein. In diesem Sinne sind die folgenden Figuren und Beispiele also nicht einschränkend auszulegen.

Erfindungsgemäße Vorrichtungen können im Übrigen in verschiedenen Größen und Füllstoffmengen in den jeweiligen Hohlräumen vorgesehen sein. Die Abmessungen der jeweiligen Hohlräume sind auf die darin jeweils enthaltene Menge an Füllstoff abgestimmt. Die Vorrichtungen wie auch die Hohlräume können unabhängig voneinander rechteckig, quadratisch, rund, mehreckig sein. In der Regel besteht die Wandung der Vorrichtung aus kompostierbarem Filterpapier oder kompostierbarem Zellstoff. Entsprechende Vorrichtungen können hergestellt werden, indem Füllstoff mit Insekten vertreibenden Mitteln behandelt oder unbehandelt, an den Stellen, an denen Hohlräume vorgesehen sind, auf einen Bogen aus Wandungsmaterial platziert wird, darüber ein weiterer Bogen Wandungsmaterial angeordnet wird, und mittels Heißversiegelung in Bereichen verschlossen wird, die die Hohlräume begrenzen und in denen obere und untere Wandung kontaktiert und miteinander verbunden werden. Der Füllstoff kann auch einfach zwischen zwei Zellstoffblättern eingebracht sein, wobei die Zellstoffblätter an ihrem jeweiligen Rand und in den Bereich zwischen zwei Hohlräumen direkt aufeinander zu liegen kommen bzw. miteinander verbunden bzw. verpresst sind, beispielsweise wie bei sogenannten Pads für Kaffeemaschinen, und ggf. zugleich verklebt oder heißversiegelt sind.

### Im Folgenden wird die Erfindung anhand von Figuren und Beispielen näher beschrieben. Es zeigen

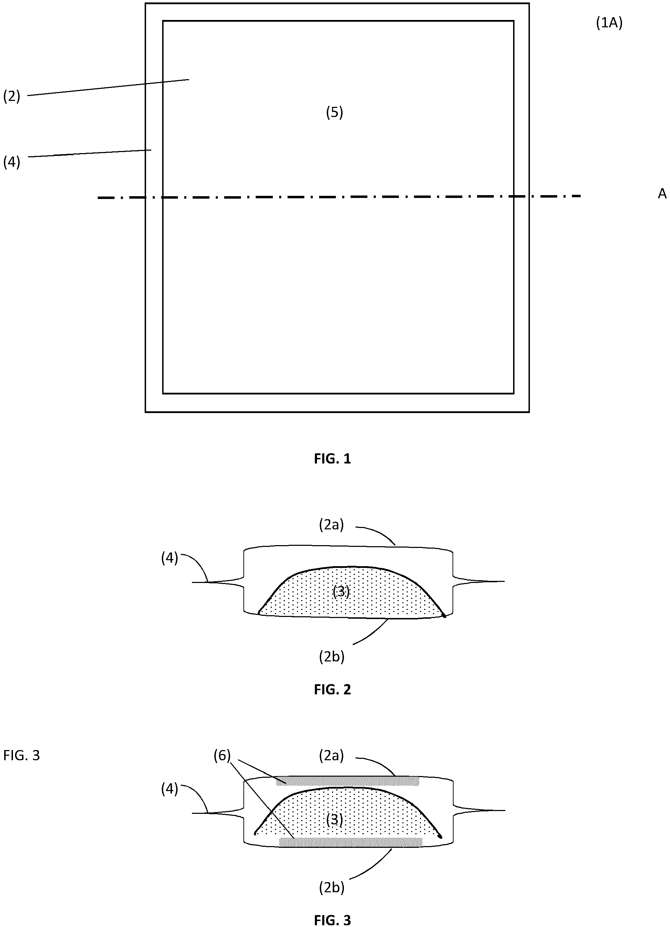

Fig. 1 eine schematische Draufsicht auf eine Vergleichsvorrichtung (1A) mit einer Wandung (2), hier in rechteckiger bzw. quadratischer Form mit nur einem Hohlraum (5) und einem Bereich (4), der den Hohlraum (5) begrenzt;
Fig. 2 eine schematische Querschnittsansicht der Vergleichsvorrichtung (1A) aus Fig. 1 entlang der dort gezeigten Querschnittslinie A mit einer oberen Wandung (2a), einer unteren Wandung (2b), Füllstoff (3) und einem Bereich (4), in dem die obere Wandung (2a) und die untereWandung (2b) aufeinandertreffen. Der Bereich (4) stellt zugleich den Fügebereich dar, in dem die obere und die untere Wandung beispielsweise durch Pressen bzw. Heißversiegelung entlang einer Kontakt- bzw. Fügefläche aneinander gefügt bzw. miteinander verbunden werden.
Fig. 3 eine schematische Querschnittsansicht einer weiteren Vergleichsvorrichtung (1A) mit Wandung (2) bestehend aus Wandungen (2a) und (2b) sowie Füllstoff (3), wobei in dieser Ausführungsform die Vorrichtung (1A) mit einem weiteren Füllstoff (6) versehen ist. Dieser kann wie dargestellt an die Wandung angrenzend vorliegen. Der Füllstoff (6) kann alternativ aber auch lose im Hohlraum (5) verteilt sein.

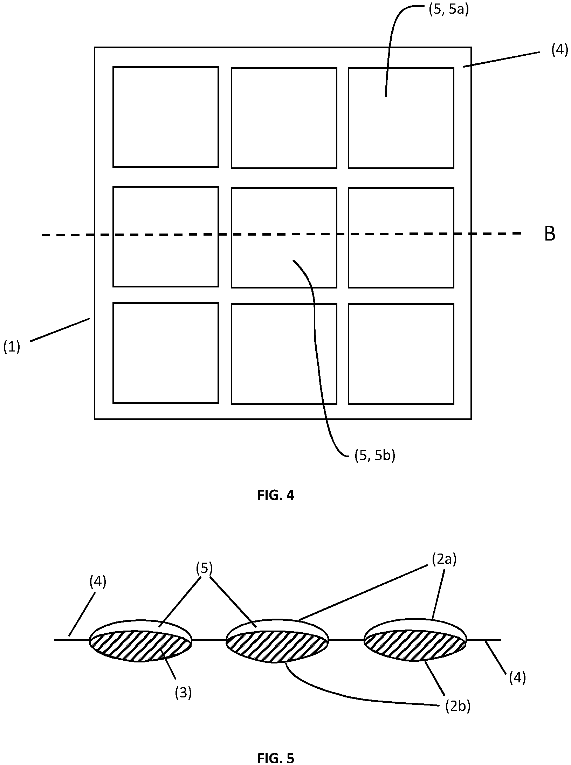

Fig. 4 eine schematische Draufsicht auf eine erfindungsgemäße Vorrichtung (1) mit Bereichen (4), in denen die obere Wandung und untere Wandung aufeinandertreffen und miteinander verbunden sind, sowie Hohlräumen (5), nämlich äußeren Hohlräumen (5a) und einem inneren Hohlraum (5b);
Fig. 5 eine schematische Querschnittsansicht der erfindungsgemäßen Vorrichtung (1) aus Figur 4 entlang der dort gezeigten Querschnittsachse B mit Bereichen (4), in denen die obere Wandung (2a) und untere Wandung (2b) aufeinandertreffen und miteinander verbunden sind, Hohlräumen (5) und Füllstoff (3);

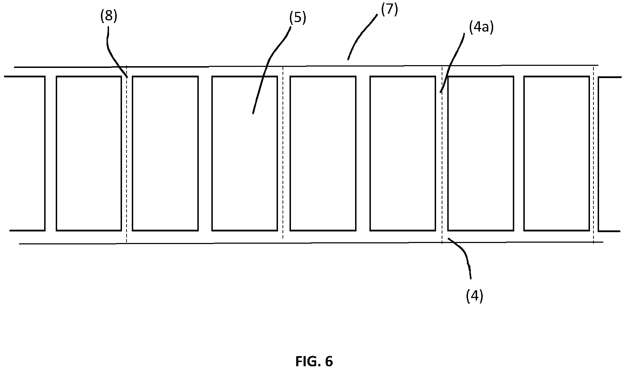

Fig. 6 eine schematische Draufsicht auf ein erfindungsgemäßes Band (7) mit einer Vielzahl von längs des Bandes aneinandergereihten Hohlräumen (5), wobei die Bereiche (4) Teilbereiche (4a) umfassen, die sich quer zur Längsrichtung des Bandes (7) erstrecken, wobei von den Teilbereichen (4a) einige mit Sollbruchstellen (8) versehen sind.

Die in den Figuren 1 bis 3 dargestellten Vergleichsvorrichtungen (1A) unterscheiden sich von erfindungsgemäßen Vorrichtungen lediglich dadurch, dass in erfindungsgemäßen Vorrichtungen mindestens zwei Hohlräume (5) vorhanden sind.

Wie in Fig. 1 dargestellt, weist eine Vergleichsvorrichtung (1A) eine Wandung (2) auf, die einen Hohlraum (5) begrenzt, wobei der Hohlraum außerdem Bereiche (4) aufweist, die den Hohlraum (5) umgeben. Fig. 2 ist eine schematische Querschnittsansicht der Vergleichsvorrichtung (1A) aus Fig. 1 entlang der dort gezeigten Querschnittslinie A. Im Querschnitt der Fig. 2 sind eine obere Wandung (2a) und eine untere Wandung (2b). In den Bereichen (4) treffen diese aufeinander und sind dort miteinander verbunden. Der Hohlraum (5) wird also begrenzt durch die Wandung (2) aus oberer Wandung (2a) und eine unterer Wandung (2b) sowie die dargestellten Bereiche (4). In dem Hohlraum (5) befindet sich ein Füllstoff (3). Die Vergleichsvorrichtung (1A) kann, wie in Fig. 3 ersichtlich, außerdem mit einem weiteren Füllstoff (6) versehen sein. Wie vorstehend bereits erwähnt kann dieser wie dargestellt an die Wandung oder Teile der Wandung angrenzend vorgesehen sein. Er kann alternativ aber auch lose im Hohlraum (5) verteilt sein.

Eine erfindungsgemäße Vorrichtung (1) ist in Fig. 4 dargestellt. Es handelt sich hierbei um eine Draufsicht auf eine erfindungsgemäße Vorrichtung (1), die in diesem Fall neun Hohlräume (5) aufweist, die jeweils quadratisch sind und in Form eines Quadrates angeordnet sind, so dass die Vorrichtung (1) einen inneren Hohlraum (5b) und acht äußere Hohlräume (5a) aufweist, wobei die Hohlräume (5) unter anderem durch Bereiche (4) begrenzt werden.

Die einzelnen Hohlräume können im Übrigen so ausgebildet sein, wie in den Vergleichsvorrichtungen (1A) der Figuren 1-3. Der Unterschied zwischen den in den Fig. 1 bis 3 gezeigten Vergleichsvorrichtungen (1A) und Vorrichtungen gemäß der Erfindung besteht lediglich in der unterschiedlichen Anzahl von Hohlräumen. Dadurch, dass erfindungsgemäße Vorrichtungen (1) mindestens zwei Hohlräume (5) aufweisen, kann der in den Hohlräumen (5) befindliche Füllstoff (3) weniger leicht verrutschen als in den Vergleichsvorrichtungen (1A).

Das ergibt sich auch aus der Querschnittsansicht der Fig. 5 einer erfindungsgemäßen Vorrichtung der Fig. 4 entlang der in Fig. 4 dargestellten Querschnittsachse B. Während in der Vergleichsvorrichtung (1A) der Fig. 1 und 2 der Füllstoff (3) in einem einzigen Hohlraum (5) untergebracht ist, sind es in der erfindungsgemäßen Vorrichtung gemäß den Fig. 4 und 5 neun Hohlräume (5), auf die sich der Füllstoff (3) insgesamt verteilt. Aus der Querschnittsansicht der Fig. 5 ist gut ersichtlich, dass der Füllstoff (3) in einer erfindungsgemäßen Vorrichtung über die Länge der Querschnittsachse B (vergl. Fig. 4, 5) in viel geringerem Maße verschiebbar ist, als in einer Vergleichsvorrichtung (1A) über die Länge der Querschnittsachse A (vergl. Fig. 1, 2).

Anhand der Fig. 4 lassen sich außerdem mehrere Vorteile einer erfindungsgemäßen Vorrichtung (1) erläutern. Dazu wird zwischen äußeren Hohlräumen (5a) und innerem Hohlraum (5b) unterschieden. Der Füllstoff (3) lässt sich grundsätzlich gleichmäßig über die Hohlräume (5) einer erfindungsgemäßen Vorrichtung (1) verteilen. Dadurch wird einem Verrutschen von Füllmittel entgegen gewirkt, wie vorstehend bereits erörtert.

Es ist ferner vorteilhaft, vorzusehen, dass sich in den äußeren Hohlräumen (5a) mehr Füllstoff (3) befindet als in inneren Hohlräumen (5b). Dadurch ändert sich die Gewichtsverteilung innerhalb der von außen betrachtet flächenförmigen Vorrichtung (1). Dies bewirkt, dass die Vorrichtung (1), wenn man sie von oben in eine Abfalltonne fallen lässt, im Wesentlichen parallel zum Boden auf dem Boden zum Aufliegen kommt. Bei einer gleichmäßigen Verteilung des Füllstoffs (3) auf alle Hohlräume (5) ist es demgegenüber häufiger dem Zufall überlassen, ob der Boden eines Abfallbehälters nach dem Einbringen der Vorrichtung (1) gleichmäßig von der Vorrichtung (1) bedeckt wird, oder nicht.

Bei der in der Fig. 4 dargestellten Vorrichtung (1) sind die Hohlräume (5) außerdem rechteckig bzw. quadratisch ausgebildet und in einem Quadrat angeordnet. Das bewirkt, dass die Vorrichtung entlang der Teile der Bereiche (4), die sich zwischen den Hohlräumen (5) parallel zum Rand der dargestellten Vorrichtung (1) erstrecken, flexibel ausgebildet ist. Abfalltonnen mit 120 l Fassungsvolumen weisen üblicherweise an ihrem Boden Rollen auf, damit man die Abfallbehälter, wenn sie befüllt sind, bequem bewegen kann. Diese Rollen befinden sich außerhalb des eigentlichen Behälters am Behälter und benötigen dort Platz, mit der Folge, dass der Boden von entsprechend ausgebildeten Abfallbehältern im Inneren über eine Stufe verfügen kann, also nicht vollständig eben ausgebildet sein muss. Lässt man nun eine Vorrichtung (1) wie in Fig. 4 dargestellt von oben auf den Boden der Tonne fallen, so kann die Vorrichtung (1) den Boden der Tonne bedecken, obwohl die vorstehend erwähnte Stufe die Bodenfläche verringert. Die in Fig. 4 dargestellte Vorrichtung (1) kann sich nämlich im Bereich der Stufe an die Form der Stufe anpassen.

Der in Fig. 3 dargestellte Querschnitt durch eine weitere Vergleichsvorrichtung (1A) verdeutlicht, dass neben dem Füllstoff (3) ein zusätzlicher Füllstoff (6) enthalten sein kann. Dabei kann es sich beispielsweise um Zellstoffwatte oder andere biologisch abbaubaren bzw. kompostierbaren und/oder industriell kompostierbaren Fasern, die zwischen der oberen Wandung (2a) bzw. der unteren Wandung (2b) und dem Füllstoff (3) angebracht ist oder lose im Pad vorliegt, handeln. Füllstoff (6) in Form von Zellstoffwatte oder anderen biologisch abbaubaren bzw. kompostierbaren und/oder industriell kompostierbaren Fasern saugt Wasser bzw. Feuchtigkeit sehr rasch auf und gibt diese weiter an den Füllstoff (3), der beispielsweise in Form von Kokoserde vorliegt, die Wasser bzw. Feuchtigkeit gut zu binden vermag. Auf die gleiche Weise kann auch ein Hohlraum in einer Vorrichtung gemäß der vorliegenden Erfindung ausgebildet sein.

Fig. 6 ist eine schematische Draufsicht auf ein erfindungsgemäßes Band (7) mit einer Vielzahl von längs des Bandes aneinandergereihten Hohlräumen (5), wobei die Bereiche (4) Teilbereiche (4a) umfassen, die sich quer zur Längsrichtung des Bandes (7) erstrecken, wobei von den Teilbereichen (4a) einige mit Sollbruchstellen (8) versehen sind.

Zwei nebeneinander liegende Hohlräume (5) auf dem Band (7) der Fig. 6 bilden dabei jeweils eine erfindungsgemäße Vorrichtung (1) mit mindestens zwei Hohlräumen (5) aus, die sich von dem Band (7) entlang der dafür vorgesehen Sollbruchstelle abtrennen lassen. Wenn keine Sollbruchstelle (8) vorgesehen ist, kann eine erfindungsgemäße Vorrichtung (1) entlang des Bereichs (4a) mit einer Schere von dem Band (7) abgetrennt werden.

Im Folgenden wird die Erfindung anhand von Ausführungsbeispielen näher erläutert. Dabei kommt der bevorzugte Füllstoff Kokoserde zum Einsatz. Wenngleich dieser Füllstoff bevorzugt ist, und seine Verwendung mit bestimmten Vorteilen einhergeht, besteht der Hauptaspekt der vorliegenden Erfindung darin, dass erfindungsgemäße Vorrichtungen mehrere Hohlräume aufweisen. Die bevorzugte Verwendung von Kokoserde ist darum nicht dahingehend auszulegen, dass als Füllstoff Kokoserde zum Einsatz kommen muss, wenngleich ihr Einsatz bevorzugt wird. Vielmehr kommen auch andere Füllstoffe in Frage, wie beispielsweise Strohpellets, da das Vorsehen von mehreren Hohlräumen auch ohne die Verwendung von Kokoserde als Füllstoff vorteilhaft ist.

Einige der Beispiele sind als Vergleichsbeispiele gekennzeichnet. In diesen kommen Vergleichsvorrichtungen (1A) zum Einsatz, wie sie in den Figuren 1 bis 3 dargestellt sind, die aus den vorgenannten Gründen aber dazu geeignet sind, den Aufbau und die Funktionsweise von einzelnen Hohlräumen (5), wie sie in erfindungsgemäßen Vorrichtungen (1) anzutreffen sind, zu erläutern.

### Ausführungsbeispiele

### Beispiele für erfindungsgemäße Vorrichtungen:

Es werden erfindungsgemäße Vorrichtungen wie folgt hergestellt:
Als Material für die Wandungen wird ein mit einem Heißversiegelungsmittel ausgerüstetes Langfaserpapier mit der Bezeichnung Dynatec (Dynatex 121/9/C) der Fa. Glatfelter verwendet, das außerdem folgende Eigenschaften aufweist:
   Ein Flächengewicht von 21,5 ± 1,5 g/m² nach ISO 536, eine Dicke von 65 ± 10 µm nach ISO 534, eine Bruchkraft längs in trockenem Zustand > 15 N/15 mm nach ISO 1924-2, eine Bruchkraft quer in trockenem Zustand > 5 N/15 mm nach ISO 1924-2, eine Bruchkraft quer in nassem Zustand > 1,4 N/15 mm gemäß Angaben des Herstellers (Glatfelter), und eine Luftdurchlässigkeit > 500 l/m²s nach EN ISO 9237.
   Von dem vorgenannten Langfaserpapier wird jeweils ein Bogen für die untere Wandung und ein Bogen für die obere Wandung verwendet. Der Bogen für die untere Wandung wird an den Stellen, an denen Hohlräume vorgesehen sind, mit Füllstoff bedeckt. Sodann wird der Bogen für die obere Wandung darüber gelegt und die beiden Bögen werden mittels einer Heißversiegelungsvorrichtung in den dafür vorgesehenen Bereichen miteinander verklebt.
   Auf diese Weise werden mehrere Vorrichtungen hergestellt, bei denen die Anzahl der Hohlräume, deren Größe, und deren Befüllung variiert.

### Beispiel 1 - für 120 l Tonne

Für eine 120 l Abfalltonne wird eine entsprechende Vorrichtung mit neun Hohlräumen mit einer Grundfläche von jeweils ca. 11 x 11 cm² hergestellt, wobei die Hohlräume so angeordnet sind, dass sich in Draufsicht auf die Vorrichtung ein Quadrat mit einem inneren und acht äußeren Hohlräumen ergibt. Die acht äußeren Hohlräume sind mit 20 bis 25 g Kokoserde gefüllt. Der innen liegende Hohlraum ist mit 10 bis 15 g Kokoserde gefüllt. Um das Volumendefizit auszugleichen, wird in dem inneren Hohlraum weniger fein gemahlene Kokoserde verwendet, als in den äußeren Hohlräumen. Entsprechende Vorrichtungen kann man von oben in eine entsprechende Abfalltonne auf den Boden fallen lassen, und der Boden wird dabei so vollständig bedeckt, wie möglich.

### Beispiel 2 - für 240 l Tonne

Für eine 240 l Abfalltonne wird eine entsprechende Vorrichtung mit 16 Hohlräumen mit einer Grundfläche von jeweils ca. 12 x 12 cm² hergestellt, wobei die Hohlräume so angeordnet sind, dass sich in Draufsicht auf die Vorrichtung ein Quadrat ergibt. Die äußeren Hohlräume sind mit jeweils 20 bis 25 g Kokoserde gefüllt. Der innen liegende Hohlraum ist mit 10 bis 15 g Kokoserde gefüllt. Um das Volumendefizit auszugleichen, wird in dem inneren Hohlraum weniger fein gemahlene Kokoserde verwendet, als in den äußeren Hohlräumen. Entsprechende Vorrichtungen kann man von oben in eine entsprechende Abfalltonne auf den Boden fallen lassen, und der Boden wird dabei so vollständig bedeckt, wie möglich.

### Beispiel 3 - für 1100 l Tonne

Für eine 1100 l Abfalltonne mit 1100 l wird eine entsprechende Vorrichtung mit 30 Hohlräumen mit einer Grundfläche von jeweils ca. 14,5 x 15,5 cm² hergestellt, wobei die Hohlräume so angeordnet sind, dass sich in Draufsicht auf die Vorrichtung ein Rechteck ergibt. Die äußeren Hohlräume sind mit jeweils 25 bis 40 g Kokoserde gefüllt. Die innen liegenden Hohlräume sind mit jeweils 15 bis 20 g Kokoserde gefüllt. Um das Volumendefizit auszugleichen, wird in den inneren Hohlräumen weniger fein gemahlene Kokoserde verwendet, als in den äußeren Hohlräumen. Entsprechende Vorrichtungen sollten zweckmäßigerweise von zwei Bedienpersonen in die Abfalltonne eingebracht werden, die die Vorrichtung gemeinsam über dem Tonnenboden ausrichten und in die Tonne fallen lassen.

### Beispiel 4 - Band mit Vorrichtungen

Auf die gleiche Weise wie vorstehend für Einzelvorrichtungen beschrieben wird ein Band ausgehend von einem unteren Band und einem oberen Band aus dem mit Heißversiegelungsmittel ausgerüsteten Langfaserpapier erzeugt, das Hohlräume mit darin befindlichem Füllstoff aufweist.

Es wurden mehrere Ausführungsformen eines entsprechenden Bandes hergestellt, bei dem die jeweiligen Hohlräume mit Kokoserde befüllt waren:

### Beispiel 4A

Ein 10 cm breites Band mit nebeneinanderliegenden Hohlräumen mit einer jeweiligen Grundfläche von ca. 5 x 10 cm², befüllt mit ca. 5 g Kokoserde, wobei die Längsseite der jeweiligen Hohlräume parallel zur Querseite und orthogonal zur Längsseite des Bandes ausgerichtet waren. Das resultierende Band kann aufgerollt werden. Von dem Band lassen sich einzelne Vorrichtungen abschneiden.

### Beispiel 4B

Dasselbe Band wurde außerdem noch einmal hergestellt und in regelmäßigen Abständen mit Perforationen in Teilbereichen versehen, die sich in Querrichtung zwischen zwei Hohlräumen über das Band erstrecken, so dass entlang der jeweiligen Perforation immer eine Vorrichtung mit zwei Hohlräumen von dem Band abgetrennt werden konnte.

Die von dem Band abgetrennten Vorrichtungen der Beispiele 4A und 4B mit jeweils zwei Hohlräumen eignen sich hervorragend für Bioabfallbeutel, wie sie beispielsweise in Küchen verwendet werden, um den Bioabfall zu sammeln, bevor er in eine Bioabfalltonne verbracht wird.

### Beispiel 4C

Es wurde außerdem ein 15 cm breites Band mit in Querrichtung drei nebeneinanderliegenden Hohlräumen, die jeweils eine quadratische Grundfläche von ca. 5 x 5 cm² aufwiesen, hergestellt.

Hohlräume dieser Ausführungsform können mit etwa 1,5 g Kokoserde befüllt sein. Das Band weist bauartbedingt entlang seiner Länge und orthogonal zu dieser ausgerichtet nebeneinanderliegende Reihen mit jeweils drei Hohlräumen auf, die jeweils mit Füllstoff befüllt sind. Zwischen diesen Reihen befinden sich Teilbereiche, in denen die obere Wandung mit der unteren Wandung heißversiegelt ist. Entlang dieser Teilbereiche lassen sich beliebig viele Reihen von dem Band abtrennen. Bauartbedingt erstrecken sich außerdem zwischen Hohlräumen befindliche Teilbereiche parallel zur Längsrichtung des Bandes. Auch in diesen Bereichen kann mit der Schere eine Trennung zwischen Hohlraumsegmenten vorgenommen werden. Es lassen sich also im Rahmen der gegebenen Möglichkeiten Vorrichtungen mit beliebig vielen zusammenhängenden Hohlräumen von dem Band abtrennen. Auf diese Weise kann die Größe der Vorrichtung an die Größe und/oder Form eines Abfallbehälters angepasst werden.

### Beispiel 4D

In einer Fortbildung des Beispiels 4C wurden in dem Band nach jeder dritten Reihe querliegender Hohlraumsegmente eine Perforation quer zur Längsrichtung des Bandes vorgesehen, sodass sich von dem Band quadratische Vorrichtungen mit jeweils neun Hohlräumen abtrennen lassen. Dem Benutzer bleibt es überlassen, welche Perforation er verwenden möchte. Für größere Gebinde kann er Vorrichtungen mit 18 Hohlräumen abtrennen. Für kleinere Gebinde kann er Vorrichtungen mit neun Hohlräumen abtrennen.

### Vergleichsbeispiel 1:

Auf ein quadratisch zugeschnittenes erstes Filterpapier aus Zellstoff mit den Maßen 10 cm x 10 cm werden 10 g Kokoserde mittig platziert. Darüber wird ein zweites quadratisch zugeschnittenes Filterpapier aus Zellstoff mit denselben Maßen wie das erste Filterpapier gelegt. Die beiden Filterpapiere werden an den Rändern durch Verpressen - einmal mit (A) und einmal ohne (B) Heißversiegelung - miteinander verbunden. Für die Variante mit der Heißversiegelung wurde ein mit einem Heißversiegelungsmittel ausgerüstetes Langfaserpapier mit der Bezeichnung Dynatec (Dynatex 121/9/C) der Fa. Glatfelter verwendet, das außerdem folgende Eigenschaften aufweist:
Ein Flächengewicht von 21,5 ± 1,5 g/m² nach ISO 536, eine Dicke von 65 ± 10 µm nach ISO 534, eine Bruchkraft längs in trockenem Zustand > 15 N/15 mm nach ISO 1924-2, eine Bruchkraft quer in trockenem Zustand > 5 N/15 mm nach ISO 1924-2, eine Bruchkraft quer in nassem Zustand > 1,4 N/15 mm gemäß Angaben des Herstellers (Glatfelter), und eine Luftdurchlässigkeit > 500 l/m²s nach EN ISO 9237.

Die so hergestellte Vorrichtung nach Variante (A) bzw. (B) kann bis zu 60 g Wasser aufnehmen.

Es tritt weniger Wasser offen aus, die Geruchsbelästigung vermindert sich, und Fliegen fühlen sich von einem entsprechend ausgestatteten Müllbeutel trotz darin vorhandenen Mülls weniger angezogen, als ein vergleichbarer Müllbeutel ohne entsprechende Vorrichtung.

Allerdings muss die Vorrichtung nach Variante (B) vorsichtig gehandhabt werden. Insbesondere kann sie bei etwas ruppiger Handhabung an den Pressrändern viel leichter aufreißen als eine Vorrichtung nach Variante (A).

### Vergleichsbeispiel 2:

Erneut wird eine Variante (A) mit und eine Variante (B) ohne Heißversiegelung hergestellt wie folgt:
   Auf ein quadratisch zugeschnittenes erstes Blatt Filterpapier mit den Maßen 30 cm x 30 cm wird eine Lage ungebleichte Zellstoffwatte mit den gleichen Maßen und einem Gewicht von 10 g gelegt. Darauf werden 30 g des geriebenen Kokoserdeziegels mittig platziert. Die Kokoserde wird mitGeraniol behandelt. Darüber wird eine zweite quadratisch zugeschnittene Lage aus Zellstoffwatte, sowie ein zweites quadratisch zugeschnittenes Blatt Filterpapier mit den gleichen Maßen wie das erste Blatt gelegt. Die beiden äußersten Blätter werden an den Rändern miteinander verbunden. Für die Variante mit der Heißversiegelung wurde ein mit einem Heißversiegelungsmittel ausgerüstetes Langfaserpapier mit der Bezeichnung Dynatec (Dynatex 121/9/C) der Fa. Glatfelter verwendet, das außerdem folgende Eigenschaften aufweist:
   Ein Flächengewicht von 21,5 ± 1,5 g/m² nach ISO 536, eine Dicke von 65 ± 10 µm nach ISO 534, eine Bruchkraft längs in trockenem Zustand > 15 N/15 mm nach ISO 1924-2, eine Bruchkraft quer in trockenem Zustand > 5 N/15 mm nach ISO 1924-2, eine Bruchkraft quer in nassem Zustand > 1,4 N/15 mm gemäß Angaben des Herstellers (Glatfelter), und eine Luftdurchlässigkeit > 500 l/m²s nach EN ISO 9237.

Die so hergestellte Vorrichtung kann bis zu 400 g Wasser aufnehmen. In einer sogenannten Biotonne, d. h. einem Sammelbehälter mit einem Volumen von ca. 120-300 l platziert wirkt die Vorrichtung einer erhöhten Geruchsbelästigung, einer erhöhten Schimmelbildung, und bei tiefen Temperaturen einem Einfrieren wirksam entgegen.

Variante (A) ist dabei deutlich weniger anfällig gegenüber einem unbeabsichtigten Aufreißen bei der Handhabung als die Variante (B).

### Vergleichsversuche zur Wasseraufnahmefähigkeit

### Vergleichsversuch 1

Durch Raspeln eines handelsüblichen Kokoserdeziegels wird Kokoserde bereitgestellt. In einem ersten Behälter A werden 10,11 g Kokoserde bei 23,4 °C mit 50,14 g Wasser versetzt. Zugleich werden in einem zweiten Behälter B 10,09 g handelsübliche Strohpellets bei 23,4 °C mit 50,07 g Wasser versetzt. Das Quellverhalten der geraspelten Kokoserde sowie der Strohpellets im jeweiligen Behälter wird beobachtet. Es wird festgestellt, dass das Wasser in Behälter A vergleichsweise rasch und nahezu vollständig von der Kokoserde aufgenommen wird. In Behälter B wird das Wasser im gleichen Zeitraum kaum oder in nur vergleichsweise geringem Umfang aufgesaugt, so dass ein deutlicher Wasserspiegel sichtbar bleibt. In Behälter B lässt sich die wässrige Phase sehr deutlich von den darin befindlichen Strohpellets (feste Phase) unterscheiden. Die Strohpellets nehmen das Wasser nur vergleichsweise langsam auf. Sie bleiben in ihrer Form erhalten. Nach 5 Minuten wird der Versuch abgebrochen. Beim Versuch, die wässrige Phase durch Dekantieren abzutrennen, lassen sich aus Behälter B 18,92 g Wasser abtrennen. Aus Behälter A lässt sich hingegen keine messbare Wassermenge abdekantieren.

### Vergleichsversuch 2

10,01 g Kokoserde wie in Versuch 1 beschrieben werden unter denselben Bedingungen wie in Versuch 1 beschrieben mit zunächst 50,04 g Wasser versetzt. Da die Wasseraufnahmefähigkeit der Kokoserde noch nicht erschöpft ist, wird weiteres Wasser hinzugegeben. Auch bei Zugabe von insgesamt 70,86 g Wasser ist die Wasseraufnahmefähigkeit nicht erschöpft. Erst bei Zugabe von insgesamt 91,36 g Wasser wird die Aufnahmefähigkeit der Kokoserde überschritten.

Die Wasseraufnahmefähigkeit von 10,16 g handelsüblicher Strohpellets wird demgegenüber bei 30,66 g Wasser erreicht, und bei insgesamt 54,79 g Wasser stark überschritten.

### Vergleichsversuch 3

Die Wasseraufnahmefähigkeit von Watte, Kokoserde und Watte mit Kokoserde wird getestet, indem nacheinander 2 g Watte, 2 g Kokoserde und eine Kombination von 1 g Kokoserde und 1 g Watte jeweils mit 20 g Wasser versetzt werden. Die verbleibende Restflüssigkeit entspricht der jeweils nicht aufgesogenen Menge an Flüssigkeit:

| | |
|---|---|
| Watte: | 2 g |
| Wasserzugabe | 20 g |
| Restflüssigkeit: | 7,87 g |
| | |
| Kokoserde: | 2 g |
| Wasserzugabe: | 20 g |
| Restflüssigkeit: | 6,38 g |
| | |
| Watte und Kokoserde: | 1 g und 1 g |
| Wasserzugabe: | 20 g |
| Restflüssigkeit: | nicht messbar |

Eine Kombination von Watte und Kokoserde weist eine synergistische Wirkung dahingehend auf, dass weniger Restflüssigkeit verbleibt, als in Anbetracht der Werte für Watte und Kokoserde alleine betrachtet zu erwarten gewesen wäre.

Vorstehend wurde die Erfindung in Hinblick auf spezifische Ausführungsformen und/oder unter Anführung bestimmter Ausführungsbeispiele beschrieben. Die vorbeschriebenen Ausführungsformen und/oder Ausführungsbeispiele sind jedoch nicht einschränkend auszulegen. Insbesondere können
- andere Füllstoffe als Kokoserde verwendet werden,
- andere Füllstoffmengen als vorstehend beschrieben eingesetzt werden,
- anstelle von Filterpapier andere wasserdurchlässige Materialien, etwa aus Stoff, Vlies oder Filz oder aus einem Netz aus biologisch abbaubaren, synthetischem und heißsiegelfähigem Kunststoff bestehende Materialien zur Herstellung des Pad- oder Kissenkörpers eingesetzt werden, und/oder
- andere Größen und Formen der Vorrichtung, als oben beschrieben, hergestellt werden,
- andere Größen, Formen und Anordnungen von Hohlräumen, als oben beschrieben, vorgesehen werden,
- andere biologisch abbaubare bzw. kompostierbare und/oder industriell kompostierbare Fasern als Zellstoffwatte verwendet werden,
- anstelle von oder zusätzlich zu naturidentischen oder natürlichen Repellentien, nicht natürliche Repellentien eingesetzt werden.

### Bezugszeichenliste

- (1A): Vergleichsvorrichtung
- (1): Vorrichtung,
- (2): Wandung,
- (2a): obere Wandung,
- (2b): untere Wandung,
- (3): Füllstoff,
- (4): Bereich,
- (4a): Teilbereich
- (5): Hohlraum,
- (5a): äußerer Hohlraum,
- (5b): innerer Hohlraum,
- (6): weiterer Füllstoff,
- (7): Band,
- (8): Sollbruchstelle.

## Patentansprüche

1. Kompostierbare, feuchtigkeitsbindende und geruchshemmende Vorrichtung (1) mit einem Feuchtigkeit aufnehmenden Füllstoff (3) zur Verwendung als Einlage in Abfallbehältern, aufweisend:
- Eine für Feuchtigkeit durchlässige Wandung (2) bestehend aus einer oberen Wandung (2a) und einer unteren Wandung (2b),
- Bereiche (4), in denen die obere Wandung (2a) und untere Wandung (2b) aufeinandertreffen und miteinander verbunden sind,
- zwischen den Bereichen (4) befindliche Hohlräume (5) mit darin befindlichem Füllstoff (3), dergestalt, dass die Hohlräume (5) mit dem darin befindlichem Füllstoff (3) durch die sie unmittelbar umgebenden Bereiche (4), in denen die obere (2a) und untere Wandung (2b) aufeinandertreffen und miteinander verbunden sind, jeweils begrenzt werden, wobei die Vorrichtung (1) mindestens zwei Hohlräume (5) mit darin befindlichem Füllstoff (3) aufweist, und die Hohlräume in einer Ebene liegen.

2. Vorrichtung (1) gemäß Anspruch 1, **dadurch gekennzeichnet, dass** der Füllstoff (3) mindestens 10%, bevorzugt mindestens 20%, bevorzugter mindestens 50%, noch bevorzugter mindestens 70%, am meisten bevorzugt mindestens 80% Kokoserde aufweist, und am allermeisten bevorzugt im Wesentlichen aus Kokoserde ist.

3. Vorrichtung (1) gemäß Anspruch 1 oder 2, **dadurch gekennzeichnet, dass** die obere Wandung (2a) und die untere Wandung (2b) in den Bereichen (4) mittels Heißversiegelung oder Kleben miteinander verbunden sind.

4. Vorrichtung (1) gemäß einem der vorhergehenden Ansprüche, **dadurch gekennzeichnet, dass** der Füllstoff (3) und/oder die obere Wandung (2a) und/oder die untere Wandung (2b) mit einem Insekten abwehrenden Mittel, bevorzugt einem natürlichen oder naturidentischen Duftstoff, besonders bevorzugt Geraniol, behandelt ist bzw. sind.

5. Vorrichtung (1) gemäß einem der vorhergehenden Ansprüche, wobei die Vorrichtung in jedem Hohlraum (5) mindestens 1, 1,5, 5, 10, 20, 30, 40 oder 50 g Füllstoff aufweist.

6. Vorrichtung (1) gemäß einem der vorhergehenden Ansprüche, **dadurch gekennzeichnet, dass** die obere Wandung (2a) und/oder die untere Wandung (2b) aus einem Material besteht, dass aus einem oder mehreren der folgenden ausgewählt ist: Filterpapier, Vlies, Stoff, Filz, oder aus einem Netz aus biologisch abbaubarem, synthetischem und heißsiegelfähigem Kunststoff.

7. Vorrichtung (1) gemäß einem der vorhergehenden Ansprüche, **dadurch gekennzeichnet, dass** die obere Wandung (2a) und/oder die untere Wandung (2b) aus einem Material besteht, dass aus einem langfaserigen Filterpapier besteht, dass mit einem biologisch abbaubarem Heißversiegelungsmittel, bevorzugt PLA, ausgerüstet ist.

8. Vorrichtung (1) gemäß einem der vorhergehenden Ansprüche, **dadurch gekennzeichnet, dass** die Vorrichtung in den Hohlräumen (5) einen weiteren Feuchtigkeit aufnehmenden Füllstoff (6) in Form von Zellstoffwatte und/oder einem anderen biologisch abbaubarem bzw. kompostierbarem Material enthält.

9. Vorrichtung (1) gemäß einem der vorhergehenden Ansprüche, wobei die Vorrichtung zwei bis 30, zwei bis 16, oder zwei bis 9 Hohlräume (5) aufweist.

10. Vorrichtung (1) gemäß einem der vorhergehenden Ansprüche, wobei die Vorrichtung an ihrem Rand eine höhere Flächendichte aufweist, als in ihrem Innenbereich.

11. Band (7) mit einer Vielzahl von längs des Bandes (7) aneinander gereihten Vorrichtungen (1) gemäß einem der vorhergehenden Ansprüche, wobei die Bereiche (4) Teilbereiche (4a) umfassen, die sich quer zur Längsrichtung des Bandes (7) erstrecken.

12. Band (7) gemäß Anspruch 11, wobei die sich quer zur Längsseite des Bandes (7) erstreckenden Teilbereiche (4a) und gegebenenfalls weitere zwischen Hohlräumen (5) befindliche Bereiche (4) mit Sollbruchstellen (8) versehen sind.

13. Verfahren zur Vermeidung von übermäßiger Geruchsbildung und/oder Bindung von Feuchtigkeit in einem Abfallbehälter, bei dem der Abfallbehälter mit einer Vorrichtung (1) gemäß einem der Ansprüche 1 bis 10 ausgestattet wird bzw. ist.

14. Abfallbehälter, ausgestattet mit einer Vorrichtung (1) gemäß einem oder mehreren der Ansprüche 1 bis 10.

15. Abfallbehälter gemäß Anspruch 14, wobei die Vorrichtung (1) an einer beliebigen Wandung, an einer Bodenwandung und/oder an einer seitlichen Wandung des Abfallbehälters befestigt ist.
